(19) Europäisches Patentamt
European Patent Office
Office européen des brevets

(11) **EP 4 278 973 A1**

(12) **EUROPEAN PATENT APPLICATION**
published in accordance with Art. 153(4) EPC

(43) Date of publication:
**22.11.2023 Bulletin 2023/47**

(21) Application number: **21919652.4**

(22) Date of filing: **15.12.2021**

(51) International Patent Classification (IPC):
**A61B 5/16** (2006.01)

(52) Cooperative Patent Classification (CPC):
**A61B 5/16**

(86) International application number:
**PCT/JP2021/046169**

(87) International publication number:
**WO 2022/153764 (21.07.2022 Gazette 2022/29)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.01.2021  JP 2021005751**

(71) Applicant: **Sony Group Corporation
Tokyo 108-0075 (JP)**

(72) Inventor: **HYODO, Yasuhide
Tokyo 108-0075 (JP)**

(74) Representative: **D Young & Co LLP
120 Holborn
London EC1N 2DY (GB)**

(54) **BIOLOGICAL INFORMATION PROCESSING DEVICE, BIOLOGICAL INFORMATION
PROCESSING SYSTEM, AND BIOLOGICAL INFORMATION PROCESSING METHOD**

(57)    A biological information processing device, a biological information processing system, and a biological information processing method which are capable of obtaining features of signals detected from a living body in real time are provided.

The present technology provides a biological information processing device including an extraction unit that extracts a component signal corresponding to a physiological index from signals detected from a living body, and a variation emphasizing unit that changes the value of the component signal, based on a time constant when the value of the component signal varies, in which the variation emphasizing unit changes a value of a first component signal in accordance with a value of a second component signal having a time constant shorter than that of the first component signal of which the value is changed. The present technology also provides a biological information processing system and a biological information processing method.

Fig. 1

**Description**

[Technical Field]

The present technology relates to a biological information processing device, a biological information processing system, and a biological information processing method.

[Background Art]

It is known that when the psychological state of a person varies, signals are transmitted from the brain via the autonomic nervous system, causing variations in functions such as respiration, skin temperature, perspiration, heart function, and vascular activity. In the related art, various physiological indexes have been used to ascertain the psychological state of a person, an animal, or the like. As an example, physiological indexes such as low frequency (LF), high frequency (HF), and pNN50 are used to grasp the activity state of the heart. In addition, physiological indexes such as a skin conductance response (SCR) and a skin conductance level (SCL) are used to grasp the state of perspiration.

Some of signals corresponding to these physiological indexes have long time constants for variation, and thus require a long measurement time to obtain features of a variation. For this reason, there is a problem that it is difficult to grasp the psychological state of a person and an animal in real time. As research on this problem, for example, NPL 1, PTL 1, and the like have been disclosed.

[Citation List]

[Non Patent Literature]

[NPL 1]
Fabiano, Diego, and Shaun Canavan. "Emotion Recognition Using Fused Physiological Signals." 2019 8th International Conference on Affective Computing and Intelligent Interaction (ACII). IEEE, 2019.

[Patent Literature]

[PTL 1]

[Summary]

[Technical Problem]

However, the technology disclosed in NPL 1 does not capture the properties of each physiological index, and thus there is a problem that obtained information cannot be sufficiently utilized. In addition, the technology disclosed in PTL 1 analyzes a delayed signal which has been delayed by a predetermined time, and thus there is a problem that features of a detected signal cannot be obtained in real time.

Consequently, a main object of the present technology is to provide a biological information processing device, a biological information processing system, and a biological information processing method which are capable of obtaining features of signals detected from a living body in real time.

[Solution to Problem]

The present technology provides a biological information processing device including an extraction unit that extracts a component signal corresponding to a physiological index from signals detected from a living body, and a variation emphasizing unit that changes the value of the component signal, based on a time constant when the value of the component signal varies, in which the variation emphasizing unit changes a value of a first component signal in accordance with a value of a second component signal having a time constant shorter than that of the first component signal of which the value is changed. The biological information processing device may further include an index calculation unit that calculates a saturation index indicating a degree of saturation when the value of the component signal varies from a reference value to a maximum value, based on the value of the component signal.

The biological information processing device may further include a storage unit that stores correspondence relationship information indicating a correspondence relationship between the saturation index related to the first component signal and the saturation index related to the second component signal, in which the variation emphasizing unit may change the value of the first component signal by multiplying the value of the first component signal by a weighting coefficient obtained from the correspondence relationship information.

The storage unit may store the correspondence relationship information in which the weighting coefficient becomes larger as the value of the saturation index related to the second component signal increases when the value of the saturation index related to the first component signal is equal to or greater than the reference value and equal to or less than a threshold value.

The storage unit may store the correspondence relationship information in which the weighting coefficient becomes smaller as the value of the saturation index related to the second component signal decreases when the value of the saturation index related to the first component signal is equal to or greater than a threshold value and equal to or less than the maximum value.

The storage unit may store the correspondence relationship information in which the weighting coefficient becomes large as the value of the saturation index related to the second component signal increases when the value of the saturation index related to the first component signal is equal to or greater than the reference value and equal to or less than a first threshold value, and the weighting coefficient becomes small as the value of the saturation index related to the second component signal decreases when the value of the saturation index related to the first component signal is equal to or greater than a second threshold value and equal to or less than the maximum value.

In the biological information processing device, the value of the component signal extracted in accordance with the physiological index may be a difference from the component signal in a resting state, and the biological information processing device may further include a resting state determination unit that determines the resting state, based on body motion information obtained from a sensor and/or the signal detected from the living body.

The biological information processing device may further include an estimation unit that estimates an emotional state based on the value of the first component signal changed by the variation emphasizing unit, and the value of the second component signal.

The biological information processing device may further include a machine learning unit that correlates the value of the first component signal changed by the variation emphasizing unit, the value of the second component signal, and an estimation result of the emotional state, to causes a learning model to perform machine learning.

The estimation unit may estimate the emotional state by using the value of the first component signal, the value of the second component signal, and the learning model having been subjected to machine learning by the machine learning unit.

In addition, the present technology provides a biological information processing system including an extraction unit that extracts a component signal corresponding to a physiological index from signals detected from a living body, and a variation emphasizing unit that changes the value of the component signal, based on a time constant when the value of the component signal varies, in which the variation emphasizing unit changes a value of a first component signal in accordance with a value of a second component signal having a time constant shorter than that of the first component signal of which the value is changed. Further, the present technology provides a biological information processing method including extracting a component signal corresponding to a physiological index from signals detected from a living body, and changing a value of a first component signal in accordance with a value of a second component signal having a time constant shorter than that of the first component signal of which the value is changed, based on a time constant when the value of the component signal varies.

[Brief Description of Drawings]

[Fig. 1]

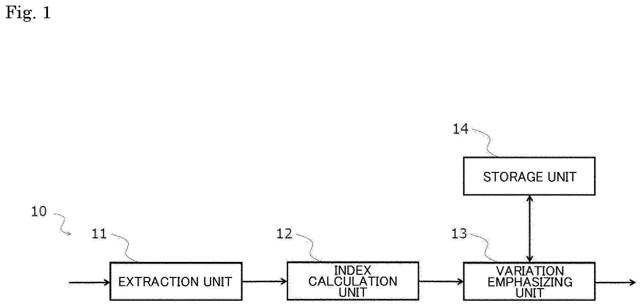

Fig. 1 is a block diagram illustrating a configuration of a biological information processing device 10 according to a first embodiment of the present technology.
[Fig. 2]

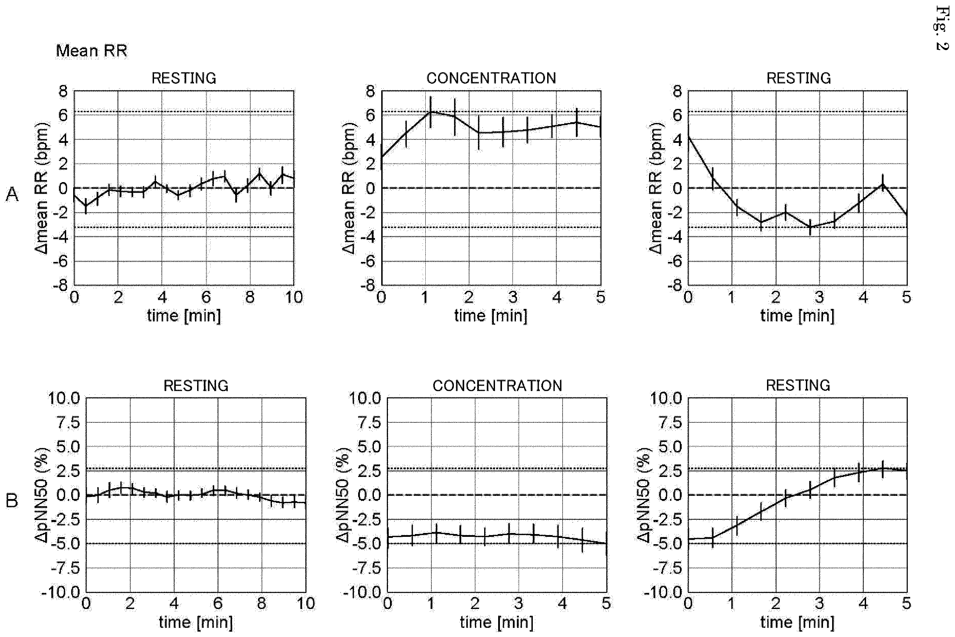

Fig. 2 is a graph showing examples of component signals obtained by the biological information processing device 10 according to an embodiment of the present technology.
[Fig. 3]

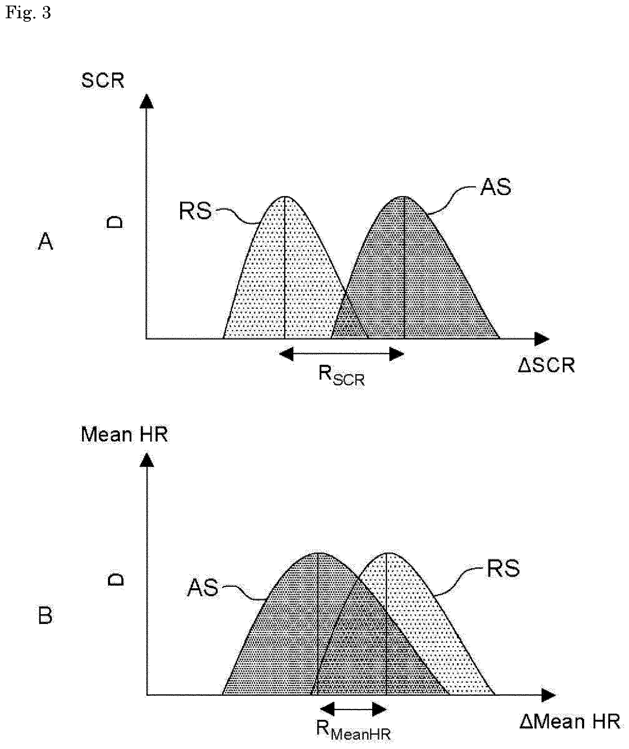

Fig. 3 is a graph showing examples of component signals obtained by the biological information processing device 10 according to the embodiment of the present technology.
[Fig. 4]

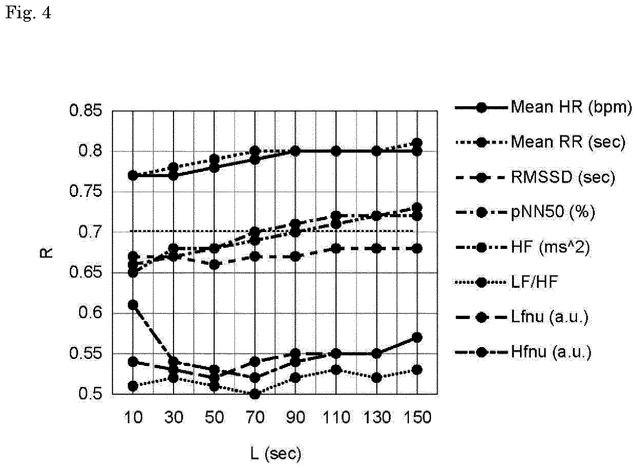

Fig. 4 is a graph showing examples of component signals obtained by the biological information processing device 10 according to the embodiment of the present technology.
[Fig. 5]

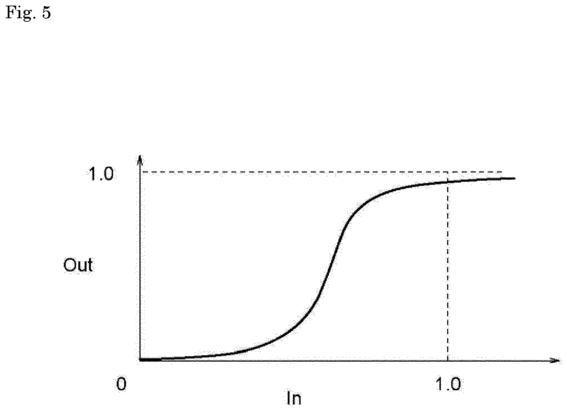

Fig. 5 is a diagram illustrating clipping performed by the biological information processing device 10 according to the embodiment of the present technology.

[Fig. 6]

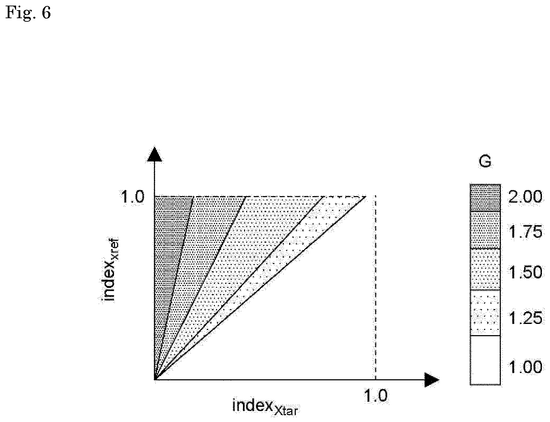

Fig. 6 is a diagram illustrating an example of correspondence relationship information according to the embodiment of the present technology.
[Fig. 7]

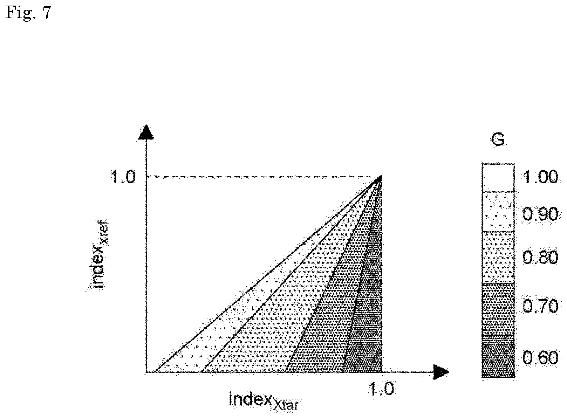

Fig. 7 is a diagram illustrating an example of correspondence relationship information according to the embodiment of the present technology.
[Fig. 8]

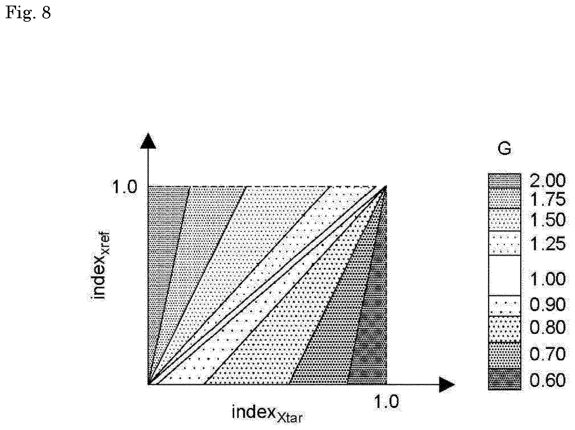

Fig. 8 is a diagram illustrating an example of correspondence relationship information according to the embodiment of the present technology.
[Fig. 9]

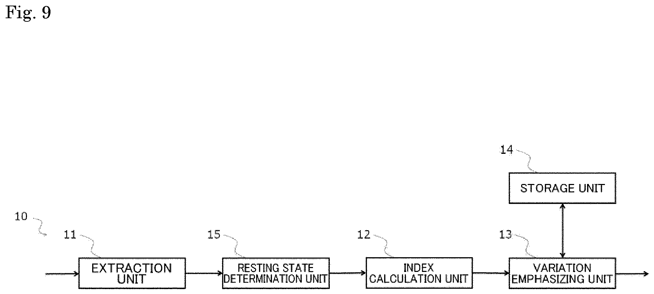

Fig. 9 is a block diagram illustrating a configuration of a biological information processing device 10 according to a second embodiment of the present technology.
[Fig. 10]

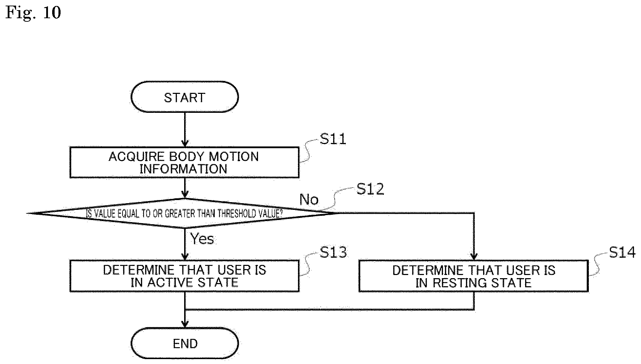

Fig. 10 is a flowchart illustrating an example of a procedure of a resting state determination unit 15 according to the embodiment of the present technology.
[Fig. 11]

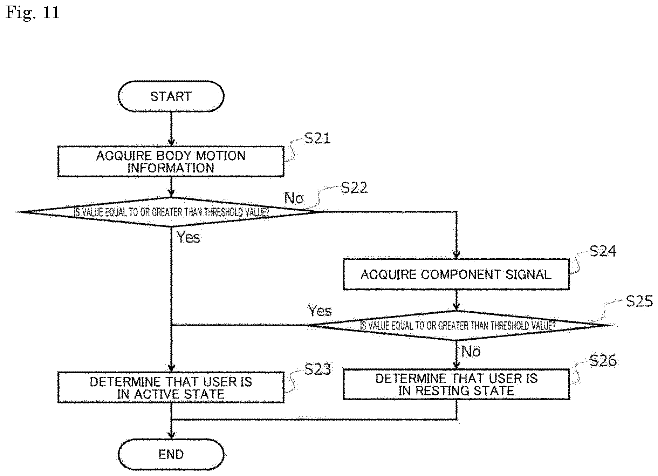

Fig. 11 is a flowchart illustrating an example of a procedure of the resting state determination unit 15 according to the embodiment of the present technology.
[Fig. 12]

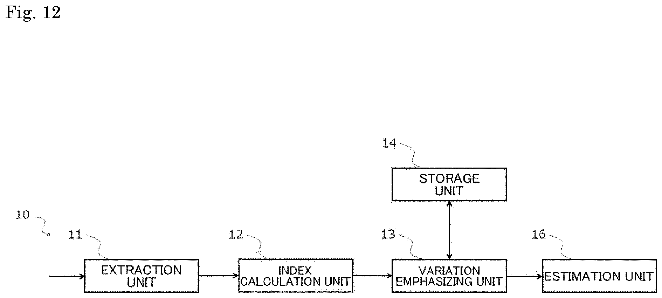

Fig. 12 is a block diagram illustrating a configuration of a biological information processing device 10 according to a third embodiment of the present technology.
[Fig. 13]

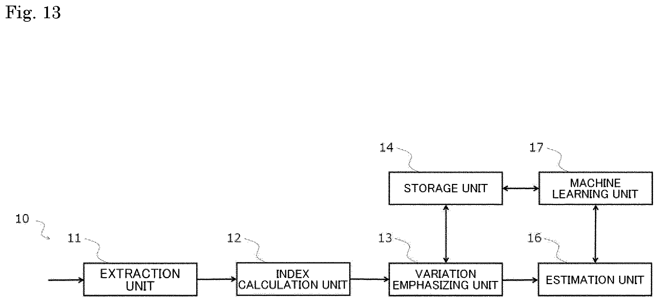

Fig. 13 is a block diagram illustrating a configuration of a biological information processing device 10 according to a fourth embodiment of the present technology.
[Fig. 14]

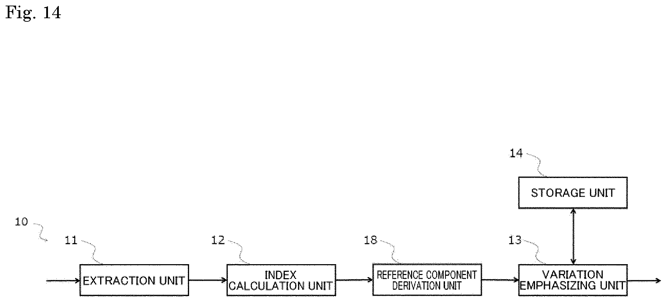

Fig. 14 is a block diagram illustrating a configuration of a biological information processing device 10 according to a fifth embodiment of the present technology.
[Fig. 15]

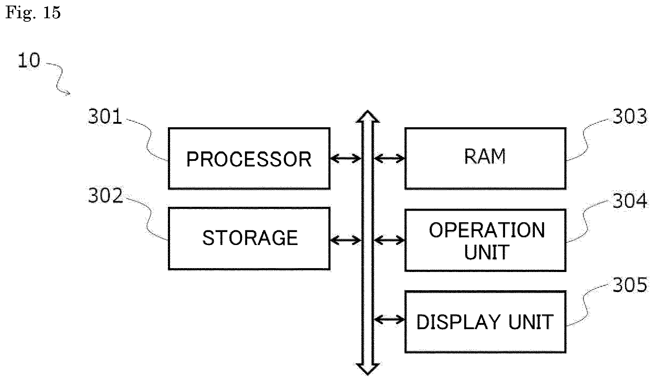

Fig. 15 is a block diagram illustrating a hardware configuration of the biological information processing device 10 according to the embodiment of the present technology.
[Fig. 16]

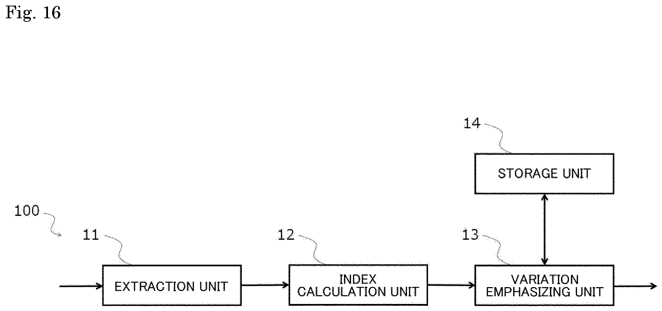

Fig. 16 is a block diagram illustrating a configuration of a biological information processing system 100 according to a sixth embodiment of the present technology.
[Fig. 17]

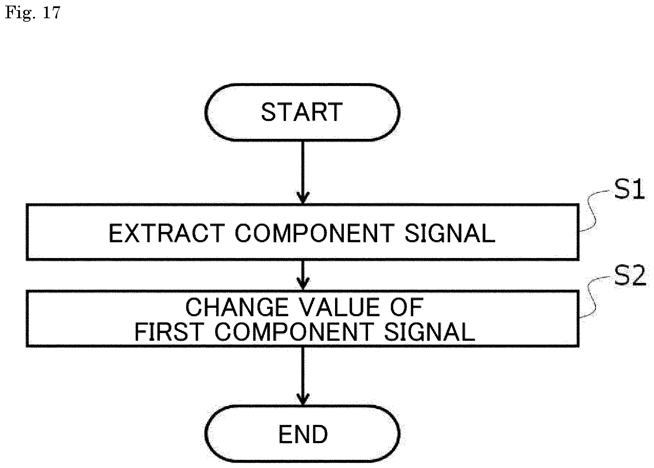

Fig. 17 is a flowchart illustrating an example of a procedure of a biological information processing method according to a seventh embodiment of the present technology.

[Description of Embodiments]

Hereinafter, preferable embodiments for implementing the present technology will be described. The following embodiments describe examples of representative embodiments of the present technology, and the scope of the present technology should not be narrowly interpreted on the basis thereof. Further, the drawings are schematic diagrams, and are not necessarily exact illustrations. In addition, the same reference signs will be applied to the same or equivalent elements or members in the drawings, and repeated description will be omitted.

The present technology will be described in the following order.

1. First embodiment of the present technology (example 1 of biological information processing device)
2. Second embodiment of the present technology (example 2 of biological information processing device)
3. Third embodiment of the present technology (example 3 of biological information processing device)
4. Fourth embodiment of the present technology (example 4 of biological information processing device)
5. Fifth embodiment of the present technology (example 5 of biological information processing device)
6. Hardware configuration
7. Sixth embodiment of the present technology (biological information processing system)
8. Seventh embodiment of the present technology (biological information processing method)

[1. First embodiment of the present technology (example 1 of biological information processing device)]

[(1) Overview]

In the related art, in order to grasp the activity state of the heart, technology for detecting signals from a living body by, for example, an electrocardiogram (ECG), photoplethysmography (PPG), and the like have been used. Further, in order to grasp the state of perspiration, technology for detecting signals from a living body by, for example, electrodermal activity (EDA) have been used. The signals include, for example, an electric signal, an optical signal, an electromagnetic wave, and the like.

Technology for grasping the psychological state of a person, an animal, or the like by extracting component signals corresponding to physiological indexes from signals detected from a living body and analyzing the component signals has been used.

As physiological indexes for grasping the psychological state of a person and an animal, for example, physiological indexes that are measured as heart rate variabilities based on the magnitudes of variations in intervals between heartbeats and represent variations are defined. Examples of physiological indexes related to a heart rate include LF, HF, LF/HF, pNN50, RMSSD, and the like.

In addition, perspiration (mental perspiration) can be electrically measured as a change in a conductance value of the skin. This is referred to as skin conductance. Examples of physiological indexes extracted from skin conductance signals include SCR that represents an instantaneous perspiration activity, SCL that represents gradual variations in skin surface conditions, and the like.

It is known that component signals corresponding to respective physiological indexes have different lengths of time constants. That is, it is known that it takes a long time to obtain a feature amount of variation from some component signals, while a short time is taken for others. This will be described with reference to Fig. 2. Fig. 2 is a graph showing an example of component signals obtained by a biological information processing device 10 according to an embodiment of the present technology. The vertical axis of the graph represents the value of a component signal, and the horizontal axis represents an elapsed time. Note that the value of the component signal on the vertical axis is a difference from a component signal in a resting state, and thus a symbol $\Delta$ is attached to the name on the vertical axis (the same applies hereinafter).

Fig. 2A illustrates variations in component signals according to a physiological index Mean RR. Fig. 2B illustrates variations in component signals according to a physiological index pNNM50. In Figs. 2A and 2B, left diagrams illustrate component signals in a determination reference state (resting state). Middle diagrams illustrate variations in component signals in an awakened state (concentration state). The awakened state refers to a state in which heartbeat, perspiration, and the like are active in response to variations in psychological state. Right diagrams illustrate variations in component signals in a resting state. The resting state refers to a state in which heartbeat, perspiration, and the like are not active.

It is known that the value of each component signal increases or decreases depending on a person's psychological state. For example, the value of pNN50, which is an index of vagal tone intensity related to heartbeat, tends to decrease when the psychological state changes from a resting state to an awakened state.

Comparing Figs. 2A and 2B, when the psychological state has changed from an awakened state (see the middle diagrams) to the resting state (see the right diagrams), a period of time until a rise of the component signal in Fig. 2B ends is longer than a period of time until a fall of the component signal in Fig. 2A ends. That is, a time constant of the component signal corresponding to the physiological index pNNM50 is longer than a time constant of the component signal corresponding to the physiological index Mean RR. From this, it can be understood that it takes a long time to obtain features of a variation in the component signal corresponding to the physiological index Mean RR.

Adverse effects caused by a long time constant will be further described with reference to Fig. 3. Fig. 3 is a graph showing an example of component signals obtained by the biological information processing device 10 according to the embodiment of the present technology. Fig. 3 illustrates an example of a component signal distribution in a resting state RS and an example of a component signal distribution in an awakened state AS.

A vertical axis D of the graph represents a probability density of component signals. The probability density is obtained by aggregating sampling data of a time-series graph as illustrated in Fig. 2.

The horizontal axis of the graph represents the value of a component signal. Note that, same as in Fig. 2, the value of a component signal is a difference from a resting state, and thus a symbol $\Delta$ is attached to the name on the vertical axis.

The highest probability density D indicates that there is a largest amount of sampling data. For this reason, the value of a component signal in the case of the highest probability density D can be zero.

Fig. 3A is a graph showing an example of a component signal distribution corresponding to a physiological index SCR. Since a component signal corresponding to the physiological index SCR has a short time constant, a portion where the resting state RS and the awakened state AS overlap is small. In other words, a distance $R_{SCR}$ between the peak of the probability density D of the resting state RS and the probability density D of the awakened state AS is long.

For this reason, it is easy to distinguish between the resting state RS and the awakened state AS.

Fig. 3B is a graph showing an example of a component signal distribution corresponding to the physiological index Mean HR. Since a component signal corresponding to the physiological index Mean HR has a long time constant, a portion where the resting state RS and the awakened state AS overlap is large. In other words, a distance $R_{MeanHR}$ between the peak of the probability density D of the resting state RS and the probability density D of the awakened state AS is short. For this reason, it is difficult to distinguish between the resting state RS and the awakened state AS.

Technology for grasping the psychological state of a person, an animal, or the like is expected to be used in wearable devices that can be worn on the hand, head, or the like and used in daily life so that the psychological state can be grasped in real time. However, when it takes a long time to obtain features of a variation in a component signal, there is a problem that the psychological state of a person, an animal, or the like cannot be grasped in real time.

[(2) Description of first embodiment]

A biological information processing device according to an embodiment of the present technology can obtain features of signals detected from a living body in real time. Thereby, it is possible to grasp the psychological state of a person, an animal, or the like in real time.

The present technology provides a biological information processing device including an extraction unit that extracts a component signal corresponding to a physiological index from signals detected from a living body, and a variation emphasizing unit that changes the value of the component signal, based on a time constant when the value of the component signal varies, in which the variation emphasizing unit changes a value of a first component signal in accordance with a value of a second component signal having a time constant shorter than that of the first component signal of which the value is changed.

A configuration of a biological information processing device 10 according to a first embodiment of the present technology will be described with reference to Fig. 1. Fig. 1 is a block diagram illustrating the configuration of the biological information processing device 10 according to the first embodiment of the present technology. As illustrated in Fig. 1, the biological information processing device 10 according to the first embodiment of the present technology includes an extraction unit 11, an index calculation unit 12, a variation emphasizing unit 13, and a storage unit 14.

The extraction unit 11 extracts a component signal corresponding to a physiological index from signals detected from a living body. The variation emphasizing unit 13 changes a value based on a time constant when the value of the component signal varies. In particular, the variation emphasizing unit 13 changes the value of a first component signal in accordance with the value of a second component signal having a time constant shorter than that of the first component signal of which the value is changed. That is, the variation emphasizing unit 13 emphasizes the feature when the value of the first component signal varies according to the value of the second component signal.

Thereby, features of component signals are obtained in real time. As a result, the psychological state of a person, an animal, or the like can be grasped in real time. Note that this effect and effects to be described later also occur similarly in other embodiments. For this reason, repetitive description may be omitted in other embodiments.

[(3) Extraction unit]

The extraction unit 11 extracts a component signal corresponding to a physiological index from signals detected from a living body. More specifically, the extraction unit 11 observes signals detected from a living body as time-series data, and extracts a component signal corresponding to a physiological index as a feature amount.

For example, a physiological index related to heartbeat will be described with reference to Fig. 4. Fig. 4 is a graph showing an example of component signals obtained by the biological information processing device 10 according to the embodiment of the present technology. In Fig. 4, Mean HR, Mean RR, RMSSD, pNN50, HF, LF/HF, Lfnu, Hfnu, and the like are shown as examples of a physiological index related to heartbeat. The extraction unit 11 extracts component signals corresponding to such physiological indexes from signals detected from the living body. Known technology may be used for the extraction. Note that physiological indexes corresponding to the component signals extracted by the extraction unit 11 are not limited to those illustrated in Fig. 4.

In Fig. 4, a horizontal axis L represents the length of a time when the extraction unit 11 extracts a component signal. A vertical axis R represents the degree of separation of each component signal. The degree of separation indicates how much a certain peak is separated from an adjacent peak in a waveform of a signal.

A component signal extracted by the extraction unit 11 may be a difference from a component signal in a resting state. Specifically, the extraction unit 11 can calculate a difference from a component signal in a resting state, for example, by subtracting an average value of the value of a component signal in a resting state as an offset. Determination regarding whether a component signal is in a resting state will be described later.

[(4) Index calculation unit]

Since the range of values that can be taken, a physical dimension, and the like differ depending on a physiological index, it is preferable that the value of a component signal be converted into a predetermined index. Consequently, the index calculation unit 12 calculates a saturation index indicating the degree of saturation when the value of a component signal varies from a reference value to a maximum value, based on the value of the component signal. The index calculation unit 12 calculates a saturation index $I_x$ when the value of the component signal is $\Delta X$, for example, according to the following Formula (1).

[Math. 1]

$$I_X = 1.0 - \frac{r_X - |\Delta X|}{r_X} \quad \cdot \cdot \cdot (1)$$

In the above Formula (1), $r_x$ indicates a distance between an average value of sampling data in a resting state and an average value of sampling data in an awakened state. $r_x$ is a constant, which is determined in advance in accordance with a physiological index.

For example, the reference value may be zero, and the maximum value may be one. It can be understood that, as the value of the saturation index $I_x$ is closer to 1, a variation in the value of the component signal is in a stabilized state (saturated state). It can be understood that, as the saturation index $I_x$ is closer to 0, the value of the component signal is in the middle of variation. By the value of the component signal being converted into a saturation index, a uniform correspondence relationship between physiological indexes having different ranges of values that can be taken, physical dimensions, and the like is obtained.

Further, since a physiological response of a person varies depending on a situation, the value of the saturation index $I_x$ may exceed a domain which is set in advance. Consequently, for robustness with respect to the value exceeding the domain, the index calculation unit 12 can clip the value of the saturation index $I_x$ so that it falls within the range of 0 to 1.

This clipping will be described with reference to Fig. 5. Fig. 5 is a diagram illustrating clipping performed by the biological information processing device 10 according to the embodiment of the present technology. A horizontal axis In in Fig. 5 represents the value of the saturation index $I_x$ before clipping, and the vertical axis Out represents the value of the saturation index $I_x$ after clipping. The index calculation unit 12 can set the value of the saturation index $I_x$ calculated according to the above Formula (1) to the horizontal axis In of Fig. 5 to obtain the value of the saturation index $I_x$ after clipping which is represented by the vertical axis Out. Using this function causes robustness in the saturation index $I_x$. Note that, although a sigmoid function is shown in Fig. 5, the present technology is not limited thereto, and for example, a line graph or the like may be used.

[(5) Variation emphasizing unit]

The variation emphasizing unit 13 changes the value of the component signal, based on a time constant when the value of the component signal varies. In particular, the variation emphasizing unit 13 changes the value of a first component signal in accordance with the value of a second component signal having a time constant shorter than that of the first component signal of which the value is changed. The time constant of the first component signal is longer than the time constant of the second component signal.

A method of distinguishing between a physiological index with a long time constant and a physiological index with a short time constant is not particularly limited. However, for example, when a time constant is longer than a predetermined threshold value, a physiological index having the time constant may be determined to be a physiological index with a long time constant. Alternatively, for example, the storage unit 14 may store a physiological index with a long time constant and a physiological index with a short time constant. Examples of the physiological index with a long time constant include ULF, VLF, LF, HF, pNN50, RMSSD, and the like as physiological indexes related to heartbeat. Physiological indexes related to perspiration include SCL and the like.

[(6) Correspondence relationship information]

The storage unit 14 stores correspondence relationship information indicating a correspondence relationship between the saturation index related to the first component signal and the saturation index related to the second component signal. Although an embodiment of this correspondence relationship information is not particularly limited, an embodiment using, for example, a lookup table or the like may be adopted. The lookup table is a table in which a correspondence relationship between two variables is modeled. This lookup table will be described with reference to Fig. 6. Fig. 6 is a diagram illustrating an example of correspondence relationship information according to an embodiment of the present technology. A left diagram in Fig. 6 illustrates a lookup table. In this diagram, a horizontal axis represents the value of a first component signal with a long time constant, and a horizontal axis represents the value of a second component signal with a short time constant.

The first component signal and the second component signal may be related to heartbeat or may be related to perspiration. Alternatively, for example, the first component signal may be related to heartbeat, and the second component signal may be related to perspiration. Examples of the second component signal include brain waves, a heart rate, SCR, and the like.

Note that the boundary lines of weighting coefficients in the lookup table are not limited to being linear as illustrated in Fig. 6. The boundary lines may be nonlinear.

A right diagram in Fig. 6 illustrates an example of weighting coefficients determined based on the value of the first component signal and the value of the second component signal.

The variation emphasizing unit 13 changes the value of the first component signal by multiplying the value of the first component signal by a weighting coefficient obtained from the correspondence relationship information. More specifically, assuming the value of the first component signal with a long time constant to be $\Delta X_{tar}$, and the value of the second component signal with a short time constant to be $\Delta X_{ref}$, a weighting coefficient is obtained by a function $G(\Delta X_{tar}, \Delta X_{ref})$. In addition, the changed value $\Delta X'_{tar}$ of the first component signal is calculated, for example, according to the following Formula (2) or the like. Thereby, features when the value of the first component signal varies are emphasized. This emphasis may be performed when transitioning from a resting state to an awakened state, or may be performed when transitioning from an awakened state to a resting state.

[Math. 2]

$$\Delta X'_{tar} = G\left(\Delta X_{ref}, \Delta X_{tar}\right) \cdot \Delta X_{tar} \quad \cdot \cdot \cdot (2)$$

The correspondence relationship information illustrated in Fig. 6 indicates that, when the value of the saturation index $\Delta X_{tar}$ related to the first component signal is equal to or greater than a reference value (for example, 0) and equal to or less than a threshold value, a weighting coefficient becomes large as the value of the saturation index $\Delta X_{ref}$ related to the second component signal increases.

Thereby, the variation emphasizing unit 13 can emphasize a variation in the value of the first component signal immediately after the first component signal rises or falls. This correspondence relationship information can be used for a physiological index in which a rise or a fall of a component signal varies relatively slowly. When a variation in the value of the first component signal is saturated, or when the values of the first component signal and the second component signal vary similarly, the variation in the value of the first component signal is not emphasized.

Examples of physiological indexes using the correspondence relationship information illustrated in Fig. 6 include LF, HF, LF/HF, pNN50, RMSSD, and the like.

Note that the threshold value can be flexibly set in accordance with a range desired to be emphasized. The same applies to the following description.

Alternatively, the storage unit 14 may store correspondence relationship information as illustrated in Fig. 7. Fig. 7 is a diagram showing an example of correspondence relationship information according to the embodiment of the present technology. The correspondence relationship information illustrated in Fig. 7 indicates that, when the value of the saturation index $\Delta X_{tar}$ related to the first component signal is equal to or greater than the threshold and equal to or less than a maximum value (for example, 1), a weighting coefficient becomes small as the value of the saturation index $\Delta X_{ref}$ related to the second component signal decreases.

Thereby, the variation emphasizing unit 13 can emphasize a variation in the value of the first component signal when the first component signal recovers from an awakened state to a resting state. This correspondence relationship information can be used for a physiological index in which a rise or a fall of a component signal varies relatively rapidly, but the recovery from an awakened state to a resting state varies relatively slowly.

Examples of a physiological index using the correspondence relationship information illustrated in Fig. 7 include SCL, a blood pressure, and the like.

Alternatively, the storage unit 14 may store correspondence relationship information as illustrated in Fig. 8. Fig. 8 is a diagram illustrating an example of correspondence relationship information according to the embodiment of the present technology. The correspondence relationship information illustrated in Fig. 8 is a combination of the correspondence relationship information illustrated in Fig. 6 and the correspondence relationship information illustrated in Fig. 7. That is, in this correspondence relationship information, when the value of the saturation index $\Delta X_{tar}$ related to the first component signal is equal to or greater than a reference value (for example, 0) and equal to or less than a first threshold value, a weighting coefficient becomes large as the value of the saturation index $\Delta X_{ref}$ related to the second component signal increases, and when the value of the saturation index $\Delta X_{tar}$ related to the first component signal is equal to or greater than a second threshold value and equal to or less than a maximum value (for example, 1), a weighting coefficient becomes small as the value of the saturation index $\Delta X_{ref}$ related to the second component signal decreases.

Thereby, the variation emphasizing unit 13 can emphasize a variation in the value of the first component signal immediately after the first component signal rises or falls. Further, the variation emphasizing unit 13 can emphasize a variation in the value of the first component signal when the first component signal recovers from an awakened state to a resting state. This correspondence relationship information can be used for a physiological index in which a rise or a fall of a component signal varies relatively slowly, and the recovery from an awakened state to a resting state varies relatively slowly.

Examples of a physiological index using the correspondence relationship information illustrated in Fig. 8 include LF, HF, LF/HF, pNN50, RMSSD, and the like.

Note that the first threshold value and the second threshold value can be flexibly set in accordance with a range desired to be emphasized. The first threshold value and the second threshold value may be different or the same.

[2. Second embodiment of the present technology (example 2 of biological information processing device)]

A configuration of a biological information processing device 10 according to a second embodiment of the present technology will be described with reference to Fig. 9. Fig. 9 is a block diagram illustrating the configuration of the biological information processing device 10 according to the second embodiment of the present technology. As illustrated in Fig. 9, the biological information processing device 10 according to the second embodiment of the present technology further includes a resting state determination unit 15.

As described above, the value of the component signal extracted according to the physiological index may be a difference from the component signal in a resting state. The resting state determination unit 15 determines the resting state based on body motion information obtained from a sensor and/or the signal detected from the living body. Thereby, noise is reduced, and the psychological state of a person, an animal, or the like can be grasped accurately.

An example of a procedure of the resting state determination unit 15 will be described with reference to Fig. 10. Fig. 10 is a flowchart illustrating the example of the procedure of the resting state determination unit 15 according to the embodiment of the present technology. As illustrated in Fig. 10, the resting state determination unit 15 acquires body motion information obtained from a sensor (step S 11). Then, when the value of the body motion information is equal to or greater than a threshold value (step S12: Yes), the resting state determination unit 15 determines that a user is in an active state (step S13).

On the other hand, when the value of the body motion information is less than the threshold value (step S12: No), the resting state determination unit 15 determines that the user is in a resting state (step S14). At this time, the resting state determination unit 15 may update the value of a component signal in a resting state.

In this manner, the resting state determination unit 15 can determine a resting state based on the body motion information obtained from the sensor.

Further, the resting state determination unit 15 may determine a resting state based on a signal detected from a living body. This will be described with reference to Fig. 11. Fig. 11 is a flowchart illustrating an example of a procedure of the resting state determination unit 15 according to the embodiment of the present technology. As illustrated in Fig. 11, the resting state determination unit 15 acquires body motion information obtained from a sensor (step S21). When the value of the body motion information is equal to or greater than a threshold value (step S22: Yes), the resting state determination unit 15 determines that a user is in an active state (step S23).

On the other hand, when the value of the body motion information is less than the threshold value (step S22: No), the resting state determination unit 15 acquires a component signal corresponding to a physiological index, which is extracted from the signal detected from the living body (step S24). When the value related to the component signal is equal to or greater than the threshold value (step S25: Yes), the resting state determination unit 15 determines that the user is in an active state (step S23). Note that the value related to the component signal may be, for example, a maximum value, a percentile value, an average value, a median value, a most frequent value, or the like.

On the other hand, when the value related to the component signal is less than the threshold value (step S25: No), the resting state determination unit 15 determines that the user is in a resting state (step S26). At this time, the resting state determination unit 15 may update the value of the component signal in the resting state.

In this manner, the resting state determination unit 15 can determine a resting state based on the signal detected from the living body in addition to the body motion information obtained from the sensor. Note that the resting state determination unit 15 may determine the resting state based only on the signal detected from the living body without acquiring body motion information.

[3. Third embodiment of the present technology (example 3 of biological information processing device)]

A configuration of a biological information processing device 10 according to a third embodiment of the present technology will be described with reference to Fig. 12. Fig. 12 is a block diagram illustrating the configuration of the biological information processing device 10 according to the third embodiment of the present technology. As illustrated in Fig. 12, the biological information processing device 10 according to the third embodiment of the present technology further includes an estimation unit 16.

The estimation unit 16 estimates emotional state (psychological state) based on the value of the first component signal changed by the variation emphasizing unit 13 and the value of the second component signal. In particular, the estimation unit 16 estimates emotional state by inputting a vector $(\Delta X_{ref}, \Delta X_{tar})$ constituted by the value of a saturation index $\Delta X_{tar}$ related to the first component signal changed by the variation emphasizing unit 13 and the value of a saturation index $\Delta X_{ref}$ related to the second component signal.

The estimation unit 16 can estimate a person's emotional state of a person in real time by the variation emphasizing unit 13 emphasizing a variation in the value of a component signal. For example, when a heart rate, SCR, SCL, and the like rise, or when RMSSD, HF, pNN50, and the like fall, the estimation unit 16 can rapidly estimate that a person's emotional state is an awakened state even when the rise or the fall varies slowly.

[4. Fourth embodiment of the present technology (example 4 of biological information processing device)]

A configuration of a biological information processing device 10 according to a fourth embodiment of the present technology will be described with reference to Fig. 13. Fig. 13 is a block diagram illustrating the configuration of the biological information processing device 10 according to the fourth embodiment of the present technology. As illustrated in Fig. 13, the biological information processing device 10 according to the fourth embodiment of the present technology further includes a machine learning unit 17.

The machine learning unit 17 causes a learning model (not illustrated) to perform machine learning the value of the first component signal changed by the variation emphasizing unit 13, the value of the second component signal, and an estimation result of an emotional state in association with each other. In particular, the machine learning unit 17 causes the learning model to perform machine learning by supervised learning in which a vector $(\Delta X_{ref}, \Delta X_{tar})$ constituted by the value of a saturation index $\Delta X_{tar}$ related to the first component signal changed by the variation emphasizing unit 13 and the value of a saturation index $\Delta X_{ref}$ related to the second component signal is set as an input teacher signal, and the estimation result of the emotional state is set as an output teacher signal.

It is widely known that this vector $(\Delta X_{ref}, \Delta X_{tar})$ is correlated with an emotional state. The correlation is described, for example, in the following literature.

Lin Shu, 7 others, "A Review of Emotion Recognition Using Physiological Signals", [online], June 28, 2018, MDPI, [searched December 6, 2020], Internet<URL: https:// www.mdpi.com/1424-8220/18/7/2074>

A learning model can be modeled, for example, with a neural network. A weighting coefficient of the neural network is updated by supervised learning in which a vector $(\Delta X_{ref}, \Delta X_{tar})$ is set as an input teacher signal, and the estimation result of the emotional state is set as an output teacher signal.

In addition, the learning model may be modeled using, for example, decision tree learning such as ID3 or random forest, correlation rule learning, or the like. Alternatively, various types of neural networks such as an artificial neural network (ANN), a deep neural network (DNN), a convolutional neural network (CNN), and a recurrent neural network (RNN) may be used. Alternatively, genetic programming (GP), inductive logic programming (ILP), a fuzzy algorithm, an evolutionary algorithm (EA), reinforcement learning, a support vector machine (SVM), hidden marcov model (HMM) clustering, a Bayesian network, and the like may be used. Further, a combination of these techniques or a technique developed by using a deep learning technique may be used.

Note that the input teacher signal may be expressed as coordinate information of a point equivalent to an end point of a vector in a vector space of a predetermined dimension, instead of the vector.

In addition, the input teacher signal may be expressed in a predetermined format in which the degree of similarity indicating how similar component signals are to each other can be calculated. As the predetermined form in which the degree of similarity can be calculated, for example, the above-described vector may be adopted, or a distance from a reference point (for example, the origin) in a vector space to the tip of each vector may be adopted. When this distance is adopted, component signals at the same distance from the reference point cannot be distinguished from each other, but component signals at different distances from the reference point can be distinguished from each other. Further, in this case, since the degree of similarity is represented by a scalar, a processing load at the time of calculating the degree of similarity between component signals is reduced.

The estimation unit 16 estimates the emotional state using the value of the first component signal, the value of the second component signal, and the learning model (learned model) having been subjected to machine learning by the machine learning unit 17. In particular, the estimation unit 16 inputs a vector $(\Delta X_{ref}, \Delta X_{tar})$, which is constituted by the value of the saturation index $\Delta X_{tar}$ related to the first component signal and the value of the saturation index $\Delta X_{ref}$ related to the second component signal and causes the learning model to estimate the emotional state.

The estimation unit 16 can estimate a person's emotional state in real time by the variation emphasizing unit 13 emphasizing a variation in the value of a component signal. For example, when a heart rate, SCR, SCL, and the like rise, or when RMSSD, HF, pNN50, and the like fall, the estimation unit 16 can rapidly estimate that a person's emotional state is an awakened state even when a variation in the rise or fall is slow.

In addition, the estimation unit 16 can perform estimation by comprehensively considering multi-dimensional indexes due to fluctuations of a living body and the like by machine learning.

[5. Fifth embodiment of the present technology (example 5 of biological information processing device)]

A configuration of a biological information processing device 10 according to a fifth embodiment of the present technology will be described with reference to Fig. 14. Fig. 14 is a block diagram illustrating the configuration of the biological information processing device 10 according to the fifth embodiment of the present technology. As illustrated in Fig. 14, the biological information processing device 10 according to the fifth embodiment of the present technology further includes a reference component derivation unit 18.

The reference component derivation unit 18 derives a second component signal with a short time constant based on a plurality of component signals. In particular, the reference component derivation unit 18 receives an input of a feature amount vector constituted by the plurality of component signals, and derives the second component signal with a short time constant by an existing analysis method. Although this analysis method is not particularly limited, for example, short time Fourier transform (STFT), wavelet transform (WT), or the like can be used. Alternatively, blind source separation (BSS) such as principal component analysis (PCA) or independent component analysis (ICA), or the like may be used.

Thereby, the biological information processing device 10 can emphasize a variation in the value of the first component signal by using the automatically derived second component signal.

[6. Hardware configuration]

A hardware configuration of the biological information processing device 10 will be described with reference to Fig. 15. Fig. 15 is a block diagram illustrating the hardware configuration of the biological information processing device 10 according to the embodiment of the present technology. As illustrated in Fig. 15, the biological information processing device 10 can include, for example, a processor 301, a storage 302, a random access memory (RAM) 303, an operation unit 304, a display unit 305, and the like as components. Components that are known in this technical field may be adopted as these components. The respective components are connected to each other, for example, by a bus serving as a data transmission line.

The processor 301 is realized by, for example, a central processing unit (CPU), a graphics processing unit (GPU), or the like, and controls each of the components of the biological information processing device 10. The processor 301 can function as, for example, the extraction unit 11, the variation emphasizing unit 13, the storage unit 14, the index calculation unit 12, and the like. The variation emphasizing unit 13 and the like can be implemented by, for example, a program. This program can function by being read to the processor 301.

The storage 302 stores programs used by the processor 301, control data such as computation parameters, and the like. As the storage 302, for example, a hard disk drive (HDD), a solid state drive (SSD), other non-volatile memories, or the like may be adopted.

The RAM 303 temporarily stores, for example, programs and the like executed by the processor 301.

The operation unit 304 receives a user's operation. For example, a keyboard, a mouse, a trackball, a tablet, a touchpad, a stick-type pointing device, a touch panel, a joystick, or the like may be adopted as the operation unit 304.

The display unit 305 provides the user with the status of processing performed by the CPU 301 and the like as images. For example, a display or the like may be adopted as the display unit 305.

Although not illustrated in the drawing, the biological information processing device 10 may include, for example, a connection unit that connects to an external computer device and a communication unit that communicates with the external computer device. The communication unit has a function of performing data communication with the external computer device via an information communication network 400 by using communication technology such as Wi-Fi, Bluetooth (registered trademark), and long term evolution (LTE).

The biological information processing device 10 may be, for example, a PC, a server, a smartphone terminal, a tablet terminal, a mobile phone terminal, a personal digital assistant (PDA), a personal computer (PC), a portable music player, a portable game console, or a wearable terminal (head mounted display (HMD), a glasses-type HMD, a watch-type terminal, a band-type terminal, or the like).

A program that implements the variation emphasizing unit 13 and the like may be stored in a computer device or computer system other than the biological information processing device 10. In this case, the biological information processing device 10 can use a cloud service that provides the functions of this program. Examples of the cloud service include software as a service (SaaS), infrastructure as a service (IaaS), platform as a service (PaaS), and the like.

The program can be stored and supplied to the computer using various types of non-transitory computer-readable media. Non-transitory computer-readable media include various types of tangible storage media. Examples of non-transitory computer-readable media include magnetic recording media (for example, floppy disks, magnetic tapes and hard disk drives), magneto-optical recording media (for example, magneto-optical discs), a compact disc read only memory (CD-ROM), CD-R, CD-R/W, and a semiconductor memory (for example, a mask ROM, a programmable ROM (PROM), an erasable PROM (EPROM), a flash ROM, and a random access memory (RAM)). The program may also be supplied to the computer by various types of transitory computer-readable medium. Examples of the transitory computer-readable media include electrical signals, optical signals, and electromagnetic waves. The transitory computer-readable media can deliver the program to the computer via wired communication channels, such as electrical wires and optical fibers, or wireless communication channels.

Note that the technology used in the present embodiment can also be used in other embodiments to be described later. The same applies to other embodiments.

[7. Sixth embodiment of the present technology (biological information processing system)]

[(1) Description of sixth embodiment]

The present technology provides a biological information processing system including an extraction unit that extracts a component signal corresponding to a physiological index from signals detected from a living body, and a variation emphasizing unit that changes the value of the component signal, based on a time constant when the value of the component signal varies, in which the variation emphasizing unit changes a value of a first component signal in accordance with a value of a second component signal having a time constant shorter than that of the first component signal of which the value is changed. With the biological information processing system, features of signals detected from a living body can be obtained in real time.

[(2) Example of sixth embodiment (biological information processing system)]

A configuration of a biological information processing system according to an embodiment of the present technology will be described with reference to Fig. 16. Fig. 16 is a block diagram illustrating a configuration of a biological information processing system 100 according to a sixth embodiment of the present technology. As illustrated in Fig. 16, the biological information processing system 100 according to the sixth embodiment of the present technology can include an extraction unit 11 and a variation emphasizing unit 13.

The extraction unit 11 extracts a component signal corresponding to a physiological index from signals detected from a living body. The variation emphasizing unit 13 changes the value of the component signal, based on a time constant when the value of the component signal varies. In particular, the variation emphasizing unit 13 changes the value of a first component signal in accordance with the value of a second component signal having a time constant shorter than that of the first component signal of which the value is changed. The time constant of the first component signal is longer than the time constant of the second component signal.

The biological information processing system 100 can use the techniques according to the other embodiments described above. Thus, detailed description of components included in the biological information processing system 100 is omitted.

[8. Seventh embodiment of the present technology (biological information processing method)]

[(1) Description of seventh embodiment]

The present technology provides a biological information processing method including extracting a component signal corresponding to a physiological index from signals detected from a living body, and changing a value of a first component signal in accordance with a value of a second component signal having a time constant shorter than that of the first component signal of which the value is changed, based on a time constant when the value of the component signal varies. With the biological information processing method, features of signals detected from a living body can be obtained in real time.

[(2) Example of seventh embodiment (biological information processing method)]

A biological information processing method according to an embodiment of the present technology will be described with reference to Fig. 17. Fig. 17 is a flowchart illustrating an example of a procedure of a biological information processing method according to a seventh embodiment of the present technology.

As illustrated in Fig. 17, the biological information processing method according to the seventh embodiment of the present technology includes extracting a component signal corresponding to a physiological index from signals detected from a living body (step S1), and changing a value of a first component signal in accordance with a value of a second component signal having a time constant shorter than that of the first component signal of which the value is changed, based on a time constant when the value of the component signal varies (step S2).

The biological information processing method according to the present embodiment may use the techniques according to the other embodiments described above. For this reason, the techniques described in the above embodiments will not be described again here.

The biological information processing method according to the present embodiment can be implemented by using software and hardware. Specifically, the biological information processing method according to the present embodiment can be implemented, for example, by a CPU provided in the hardware reading a program for implementing the biological information processing method according to the present embodiment.

In addition to this, the configurations described in the above embodiments can be selected or changed as appropriate to other configurations as long as doing so does not depart from the essential spirit of the present technology.

The effects described in the present specification are merely illustrative and not limitative, and other effects may be obtained.

The present technology can also have the following configurations.

[1] A biological information processing device including:

an extraction unit that extracts a component signal corresponding to a physiological index from signals detected from a living body; and
a variation emphasizing unit that changes the value of the component signal, based on a time constant when the value of the component signal varies,
wherein the variation emphasizing unit changes a value of a first component signal in accordance with a value of a second component signal having a time constant shorter than that of the first component signal of which the value is changed.

[2] The biological information processing device according to [1], further including: an index calculation unit that calculates a saturation index indicating a degree of saturation when the value of the component signal varies from a reference value to a maximum value, based on the value of the component signal.

[3] The biological information processing device according to [2], further including:

a storage unit that stores correspondence relationship information indicating a correspondence relationship between the saturation index related to the first component signal and the saturation index related to the second component signal,
wherein the variation emphasizing unit changes the value of the first component signal by multiplying the value of the first component signal by a weighting coefficient obtained from the correspondence relationship information.

[4] The biological information processing device according to [3], wherein the storage unit stores the correspondence relationship information in which the weighting coefficient becomes large as the value of the saturation index related to the second component signal increases when the value of the saturation index related to the first component signal is equal to or greater than the reference value and equal to or less than a threshold value.

[5] The biological information processing device according to [3], wherein the storage unit stores the correspondence relationship information in which the weighting coefficient becomes small as the value of the saturation index related to the second component signal decreases when the value of the saturation index related to the first component signal is equal to or greater than a threshold value and equal to or less than the maximum value.

[6] The biological information processing device according to [3], wherein the storage unit stores the correspondence relationship information in which the weighting coefficient becomes large as the value of the saturation index related to the second component signal increases when the value of the saturation index related to the first component signal is equal to or greater than the reference value and equal to or less than a first threshold value, and the weighting coefficient becomes small as the value of the saturation index related to the second component signal decreases when the value of the saturation index related to the first component signal is equal to or greater than a second threshold value and equal to or less than the maximum value.

[7] The biological information processing device according to any one of [1] to [6], wherein the value of the component signal extracted in accordance with the physiological index is a difference from the component signal in a resting state, and

the biological information processing device further includes a resting state determination unit that determines the resting state, based on body motion information obtained from a sensor and/or the signal detected from the living body.

[8] The biological information processing device according to any one of [1] to [7], further including:

an estimation unit that estimates an emotional state, based on the value of the first component signal changed by the variation emphasizing unit, and the value of the second component signal.

[9] The biological information processing device according to [8], further including: a machine learning unit that correlates the value of the first component signal changed by the variation emphasizing unit, the value of the second component signal, and an estimation result of the emotional state, to causes a learning model to perform machine learning.

[10] The biological information processing device according to [8] or [9], wherein the estimation unit estimates the emotional state by using the value of the first component signal, the value of the second component signal, and the learning model having been subjected to machine learning by the machine learning unit.

[11] A biological information processing system including:

an extraction unit that extracts a component signal corresponding to a physiological index from signals detected from a living body; and

a variation emphasizing unit that changes the value of the component signal, based on a time constant when the value of the component signal varies,

wherein the variation emphasizing unit changes a value of a first component signal in accordance with a value of a second component signal having a time constant shorter than that of the first component signal of which the value is changed.

[12] A biological information processing method including:

extracting a component signal corresponding to a physiological index from signals detected from a living body; and

changing a value of a first component signal in accordance with a value of a second component signal having a time constant shorter than that of the first component signal of which the value is changed, based on a time constant when the value of the component signal varies.

[Reference Signs List]

10 Biological information processing device
11 Extraction unit
12 Index calculation unit
13 Variation emphasizing unit
14 Storage unit
15 Resting state determination unit
16 Estimation unit
17 Machine learning unit
18 Reference component derivation unit
100 Biological information processing system
S1 Extracting component signal
S2 Changing value of first component signal

**Claims**

1. A biological information processing device comprising:

an extraction unit that extracts a component signal corresponding to a physiological index from signals detected from a living body; and

a variation emphasizing unit that changes the value of the component signal, based on a time constant when the value of the component signal varies,

wherein the variation emphasizing unit changes a value of a first component signal in accordance with a value of a second component signal having a time constant shorter than that of the first component signal of which the value is changed.

2. The biological information processing device according to claim 1, further comprising:
an index calculation unit that calculates a saturation index indicating a degree of saturation when the value of the component signal varies from a reference value to a maximum value, based on the value of the component signal.

3. The biological information processing device according to claim 2, further comprising:

a storage unit that stores correspondence relationship information indicating a correspondence relationship between the saturation index related to the first component signal and the saturation index related to the second component signal,

wherein the variation emphasizing unit changes the value of the first component signal by multiplying the value of the first component signal by a weighting coefficient obtained from the correspondence relationship information.

4. The biological information processing device according to claim 3, wherein the storage unit stores the correspondence relationship information in which the weighting coefficient becomes large as the value of the saturation index related to the second component signal increases when the value of the saturation index related to the first component signal is equal to or greater than the reference value and equal to or less than a threshold value.

5. The biological information processing device according to claim 3, wherein the storage unit stores the correspondence relationship information in which the weighting coefficient becomes small as the value of the saturation index related to the second component signal decreases when the value of the saturation index related to the first component signal is equal to or greater than a threshold value and equal to or less than the maximum value.

6. The biological information processing device according to claim 3, wherein the storage unit stores the correspondence relationship information in which the weighting coefficient becomes large as the value of the saturation index related to the second component signal increases when the value of the saturation index related to the first component signal is equal to or greater than the reference value and equal to or less than a first threshold value, and the weighting coefficient becomes small as the value of the saturation index related to the second component signal decreases when the value of the saturation index related to the first component signal is equal to or greater than a second threshold value and equal to or less than the maximum value.

7. The biological information processing device according to claim 1, wherein the value of the component signal extracted in accordance with the physiological index is a difference from the component signal in a resting state, and the biological information processing device further comprises a resting state determination unit that determines the resting state, based on body motion information obtained from a sensor and/or the signal detected from the living body.

8. The biological information processing device according to claim 1, further comprising:
an estimation unit that estimates an emotional state, based on the value of the first component signal changed by the variation emphasizing unit, and the value of the second component signal.

9. The biological information processing device according to claim 8, further comprising:
a machine learning unit that correlates the value of the first component signal changed by the variation emphasizing unit, the value of the second component signal, and an estimation result of the emotional state, to causes a learning model to perform machine learning.

10. The biological information processing device according to claim 8, wherein the estimation unit estimates the emotional state by using the value of the first component signal, the value of the second component signal, and the learning model having been subjected to machine learning by the machine learning unit.

11. A biological information processing system comprising:

an extraction unit that extracts a component signal corresponding to a physiological index from signals detected from a living body; and

a variation emphasizing unit that changes the value of the component signal, based on a time constant when the value of the component signal varies,

wherein the variation emphasizing unit changes a value of a first component signal in accordance with a value of a second component signal having a time constant shorter than that of the first component signal of which the value is changed.

12. A biological information processing method comprising:

extracting a component signal corresponding to a physiological index from signals detected from a living body; and

changing a value of a first component signal in accordance with a value of a second component signal having a time constant shorter than that of the first component signal of which the value is changed, based on a time constant when the value of the component signal varies.

Fig. 1

Fig. 2

Fig. 3

Fig. 4

Fig. 5

Fig. 6

Fig. 7

Fig. 8

Fig. 9

STORAGE UNIT 14

10

11 EXTRACTION UNIT → 15 RESTING STATE DETERMINATION UNIT → 12 INDEX CALCULATION UNIT → 13 VARIATION EMPHASIZING UNIT →

Fig. 10

```
                    ┌──────────────┐
                    │    START     │
                    └──────┬───────┘
                           │
                           ▼
            ┌──────────────────────────────┐  ⌐S11
            │     ACQUIRE BODY MOTION       │
            │        INFORMATION            │
            └──────────────┬───────────────┘
                           │                        ⌐S12
                           ▼                  No
          ◇──────────────────────────────────────◇───────────┐
          ◇ IS VALUE EQUAL TO OR GREATER THAN THRESHOLD VALUE?◇           │
          ◇──────────────────────────────────────◇           │
                           │  Yes                              │
                           ▼                                   ▼
            ┌──────────────────────────┐ ⌐S13   ┌──────────────────────────┐ ⌐S14
            │   DETERMINE THAT USER IS  │        │   DETERMINE THAT USER IS  │
            │      IN ACTIVE STATE      │        │     IN RESTING STATE      │
            └──────────────┬───────────┘        └──────────────────────────┘
                           │                                   │
                           ▼◄──────────────────────────────────┘
                    ┌──────────────┐
                    │     END      │
                    └──────────────┘
```

Fig. 11

```
                    ┌──────────────┐
                    │    START     │
                    └──────┬───────┘
                           │
                           ▼
              ┌─────────────────────────┐  S21
              │  ACQUIRE BODY MOTION     │
              │      INFORMATION         │
              └────────────┬────────────┘
                           │          No      S22
                           ▼         ┌──────────────────────────┐
        ◇ IS VALUE EQUAL TO OR GREATER THAN THRESHOLD VALUE? ◇───┤
                           │                                     │
                         Yes                                     ▼
                           │                    ┌────────────────────────────┐ S24
                           │                    │  ACQUIRE COMPONENT SIGNAL   │
                           │                    └──────────────┬─────────────┘
                           │       Yes                         │
                           │◄───── ◇ IS VALUE EQUAL TO OR GREATER THAN THRESHOLD VALUE? ◇ S25
                           │                                   │
                           │                                  No
                           ▼                                   ▼
              ┌─────────────────────┐ S23         ┌─────────────────────┐ S26
              │ DETERMINE THAT USER IS│           │ DETERMINE THAT USER IS│
              │   IN ACTIVE STATE     │           │   IN RESTING STATE    │
              └──────────┬──────────┘             └──────────┬──────────┘
                         │                                   │
                         ▼                                   │
                    ┌──────────┐                             │
                    │   END    │◄────────────────────────────┘
                    └──────────┘
```

Fig. 12

Fig. 13

```
                                    ┌──────────────┐       ┌──────────────┐
                                 14 │ STORAGE UNIT │    17 │   MACHINE    │
                                    │              │◄─────►│ LEARNING UNIT│
                                    └──────┬───────┘       └──────┬───────┘
                                           │                      │
                                           ▼                      ▼
10                                         │                      │
   11 ┌────────────┐  12 ┌──────────────┐ 13 ┌──────────────┐ 16 ┌──────────────┐
 ────►│ EXTRACTION │────►│    INDEX     │───►│  VARIATION   │───►│  ESTIMATION  │
      │    UNIT    │     │CALCULATION UNIT│   │EMPHASIZING UNIT│   │     UNIT     │
      └────────────┘     └──────────────┘   └──────────────┘   └──────────────┘
```

Fig. 14

10

11

14

STORAGE UNIT

12

18

13

EXTRACTION UNIT → INDEX CALCULATION UNIT → REFERENCE COMPONENT DERIVATION UNIT → VARIATION EMPHASIZING UNIT →

Fig. 15

10

301 PROCESSOR

302 STORAGE

303 RAM

304 OPERATION UNIT

305 DISPLAY UNIT

Fig. 16

Fig. 17

```
           ┌─────────────┐
           │    START    │
           └─────────────┘
                  │
                  ▼
  ┌──────────────────────────────────────┐  ⌇ S1
  │       EXTRACT COMPONENT SIGNAL        │
  └──────────────────────────────────────┘
                  │
                  ▼
  ┌──────────────────────────────────────┐  ⌇ S2
  │           CHANGE VALUE OF             │
  │       FIRST COMPONENT SIGNAL          │
  └──────────────────────────────────────┘
                  │
                  ▼
           ┌─────────────┐
           │     END     │
           └─────────────┘
```

## INTERNATIONAL SEARCH REPORT

| International application No. |
|---|
| **PCT/JP2021/046169** |

### A. CLASSIFICATION OF SUBJECT MATTER

*A61B 5/16*(2006.01)i
FI:  A61B5/16 100

According to International Patent Classification (IPC) or to both national classification and IPC

### B. FIELDS SEARCHED

Minimum documentation searched (classification system followed by classification symbols)

A61B5/00-5/0538; A61B5/06-5/398; A61B10/00-10/06

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 3-75531 A (NIPPON STEEL CORP.) 29 March 1991 (1991-03-29) page 3, upper left column, line 10 to page 4, lower right column, line 10, fig. 1-8 | 1-2, 7, 11-12 |
| Y | | 8-10 |
| A | | 3-6 |
| Y | JP 2020-10803 A (SONY CORP.) 23 January 2020 (2020-01-23) paragraph [0011] | 8-10 |
| X | JP 2019-13750 A (ARKRAY, INC.) 31 January 2019 (2019-01-31) paragraphs [0002]-[0005], [0036], [0039]-[0042] | 12 |
| A | | 1-11 |
| X | JP 2011-62335 A (PANASONIC ELECTRIC WORKS CO., LTD.) 31 March 2011 (2011-03-31) paragraphs [0029]-[0051], fig. 1-6 | 12 |
| A | | 1-11 |
| A | JP 2007-501028 A (DEXCOM, INC) 25 January 2007 (2007-01-25) paragraphs [0344], [0345], fig. 6, 7 | 1-12 |

☐ Further documents are listed in the continuation of Box C.      ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **01 February 2022** | **15 February 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2021/046169**

| Patent document cited in search report | | | Publication date (day/month/year) | Patent family member(s) | | | Publication date (day/month/year) |
|---|---|---|---|---|---|---|---|
| JP | 3-75531 | A | 29 March 1991 | (Family: none) | | | |
| JP | 2020-10803 | A | 23 January 2020 | US<br>paragraph [0067]<br>WO<br>CN | 2021/0275103<br><br>2020/017162<br>112399823 | A1<br><br>A1<br>A | |
| JP | 2019-13750 | A | 31 January 2019 | US<br>paragraphs [0003]-[0006],<br>[0044], [0047]-[0050]<br>EP<br>CN | 2019/0011467<br><br><br>3424423<br>109211987 | A1<br><br><br>A1<br>A | |
| JP | 2011-62335 | A | 31 March 2011 | (Family: none) | | | |
| JP | 2007-501028 | A | 25 January 2007 | US<br>paragraph [0339], fig. 6, 7<br>WO<br>EP | 2005/0027180<br><br>2005/011489<br>1648293 | A1<br><br>A1<br>A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Emotion Recognition Using Fused Physiological Signals. **FABIANO ; DIEGO ; SHAUN CANAVAN.** International Conference on Affective Computing and Intelligent Interaction (ACII). IEEE, 2019

- **LIN SHU.** A Review of Emotion Recognition Using Physiological Signals. *MDPI,* 06 December 2020, https:// www.mdpi.com/1424-8220/18/7/2074>